# EUROPEAN PATENT SPECIFICATION

(11) **EP 1 674 133 B1**
(45) Date of publication and mention of the grant of the patent: **07.07.2010**
(21) Application number: 04029773.1
(22) Date of filing: 16.12.2004
(51) Int. Cl.: A61Q 5/02, A61Q 5/12, A61Q 19/10, A61K 8/35, A61K 8/362, A61K 8/365, A61K 8/44, A61K 8/49, C11D 3/20, C11D 3/33, C11D 3/40

(54) **Cleansing composition**
Reinigungsmittel
Composition de nettoyage

(43) Date of publication of application: 28.06.2006
(73) Proprietor: KPSS-Kao Professional Salon Services GmbH, 64297 Darmstadt (DE)
(72) Inventor: Molenda, Michael, 60598 Frankfurt (DE); Lateulere, Magali, 64297 Darmstadt (DE)

(56) References cited:
- EP-A- 1 166 752
- EP-A- 1 266 651
- EP-A- 1 504 749
- WO-A-00/25730
- WO-A-01/04251
- WO-A-03/103622
- US-A- 6 019 991
- US-A1- 2004 063 597
- US-A1- 2004 116 539
- US-A1- 2004 154 108
- US-B1- 6 541 436
- US-B1- 6 635 702

## Description

The present invention is related to the stabilization of color of cleansing compositions on aqueous basis, particularly body cleansing compositions such as shampoo, shower gel, face and hand cleansing compositions.

Colorful cleansing compositions have become more and more favorable in the cosmetic market. The colors serve for attractive appearance especially for transparent compositions in a transparent packaging material, as well as, from consumer point of view, helps to identify product and more specifically communicates to the consumer product benefit such as most of the anti-dandruff shampoos are colored blue.

For coloring shampoo compositions, anionic, neutral, cationic and natural dyes or their mixtures are used. Especially preferred ones are the anionic ones.

Stability of color of cleansing composition is an important issue as most of the dyes are decomposing by environmental effects and especially by irradiation to sun and UV rays. In practice, color stabilization is a major subject matter for the formulators of such composition. It has been observed throughout the years that special difficulties exist especially with blue and red dyes.

It has also been observed that the color stability problem is aggravated especially when the pH of the composition becomes more and more acidic and especially below 4.5.

The present invention starts from the problem to prevent or at least to diminish color fading - without visible colour change - or disappearing of the liquid cleansing compositions.

It has surprisingly been found out that the color of the cleansing composition comprising at least one surfactant selected from anionic, nonionic, amphoteric and zwitterionic ones is stabilized when the composition comprises ethylenediaminetetraacetic acid and/or its potassium and sodium salts and/or their mixtures, benzotriazolyl dodecyl p-cresol and has a pH between 2 and 4.

Further object of the invention is the use of a combination of ethylenediaminetetraacetic acid and/or its salts and benzotriazolyl dodecyl p-cresol for the stabilization of the color of cleansing compositions comprising at least one surfactant selected from anionic, nonionic, amphoteric and zwitterionic ones. Special attention is given to the compositions designed for hair washing, shampoo.

In practice the combination of the compounds, at least one chelating agent and at least one UV absorbing compound, stabilizes the color of liquid cleansing compositions at a wider pH range from 2 to 7. EP 1266652 A2 discloses UV filter and chelating agent combination for stabilizing colour of liquid compositions. However, the document is silent on the combination of present invention.

During the experimental studies, it has been found out that when UV absorbing agent and chelating agents are used individually, mostly color stabilization is not observed or the effect is at very low level, if present. Once again the problem is aggravated when the pH of the cleansing composition is acidic and especially below 4.5. When both compounds are contained in the same composition, color of the compositions is stabilized. This shows synergistic effects of the both components.

DE 195 15 698 deals with the stabilization of the color of liquid cleansing compositions and provides as a solution the use of a known antimicrobial agent pyrithion or its salts at a lower concentration range. The document is silent on use of any combination as provided with the present invention.

The concentration of the chelating agent is in the range of 0.01 to 5%, preferably 0.05 to 4% and more preferably 0.05 to 3% and most preferably 0.05 to 2.5% by weight calculated to the total compositions.

The amount of the UV-absorber ranges typically from 0.01 % to 2.5%, preferably from 0.05 % to 2, more preferably 0.05 to 1.5 and most preferably 0.05 to 1% % by weight, calculated to the total composition.

According to the invention, the liquid cleansing compositions are colored with anionic, neutral, cationic or natural dyestuffs or their combinations. The suitable anionic dyestuffs are any usable for cosmetic purposes and especially those available for product colouring purposes. Without limiting possibility of using other not listed ones, examples of the suitable anionic dyes are: Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

As the dyes used for colouring the composition and not having any dyeing effect on the surface of the object treated with it, their concentration is usually quite low and in any case lower than 0.25%, preferably lower than 0.2% and more preferably lower than 0.15% and most preferably lower than 0.1 % by weight calculated to the total composition. It should be noted that in the case of dark dyestuffs such as blue, red or any other darker direction the concentration may also be in the ppm range. The concentrations mentioned here are total dyestuff concentration and not refer to the individual dyes.

Although not the first choice for the purpose of product colouring, liquid-cleansing compositions may also be coloured with cationic dyes. Their substantivity to the negatively charged surfaces because of their cationic nature should be kept in mind when using those dyes. Those dyes are especially used as direct dyes in cleansing and colouring compositions for hair. Some examples again without limiting the selection are: Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

The concentration of the cationic dyes in the liquid cleansing compositions is usually very low and in any case lower than 0.05% by weight when used alone for product colouring purposes.

Additionally, the liquid cleansing compositions may also be colored with neutral dyes (HC dyes), so called nitro dyes. Here again their substantivity to the surfaces should be taken into consideration when using the dyestuffs. Hair cleansing compositions are particularly mentioned in this concern. The concentration of the neutral dyes is also low and in any case concentrations higher than 0.05% by weigh are not used for coloring liquid cleansing compositions. Some of the neutral dyes to mention, without limiting the selection or the use of the not mentioned ones are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No. 13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used for coloring liquid cleansing compositions for example henna (red or black), alkanna root, laccaic acid, indigo, logwood powder, madder root and rhubarb powder.

It should be noted that anionic, cationic, neutral and plant (natural) dyes are as well used in combination with each other.

The concentration of total dyestuffs, one or more and in mixture with any other either anionic, or cationic, or neutral or natural, is lower than 0.25%, preferably lower than 0.2% and more preferably lower than 0.15% and most preferably lower than 0.1 % by weight calculated to the total composition. As the subject matter of the present invention is colour stabilization, the composition in any case comprise one or more dyestuffs and the concentration can be as low as 0.00001 % by weight calculated to total composition.

Among all the dyestuffs disclosed above, anionic dyes are found to be the most suitable and preferred ones.

Colored liquid cleansing compositions are usually in the form of conventional liquid thickened shampoo packed or confectioned especially into a transparent packaging. Colored liquid cleansing compositions in the form of a ready to use foam are also within the scope of the present invention as well packed into either a transparent pump-foamer or into a transparent aerosol bottle. In the case that an aerosol foam preparation is preferred, propellant gas must be added to the formulation. The suitable propellant gasses are carbon dioxide, dimethylether and alkanes such as butane propane or their mixtures.

Liquid cleansing compositions comprise at least one surfactant selected from anionic, non-ionic and/or amphoteric or zwitterionic surfactants at a concentration range of 1 to 50%, preferably 5 to 40% and more preferably 5 to 30%, and most preferably 5 to 25% by weight, calculated to the total composition.

In an embodiment of the present invention, especially in the case that the composition is designed as personal cleansing composition, the liquid cleansing composition comprises at least one anionic, at least one nonionic surfactant. More preferably the compositions further comprise additionally at least one amphoteric surfactant.

Anionic surfactants are preferably present in an amount from 1 to 30%, preferably 2 to 20% and most preferably 2 - 15%, and most preferably 2 to 10% by weight, calculated to the total composition.

These are anionic surfactants of the sulfate, sulfonate, carboxylate and alkyl phosphate type, especially, of course, those customarily used in body cleansing compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful in the liquid cleansing compositions are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁ - (C₂H₄O)ₙ- O - CH₂COOX,

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 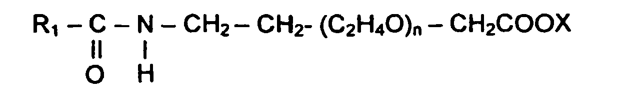 wherein R₁ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.
It is also possible to use mixtures of several anionic surfactants, for example an ether sulfate and a polyether carboxylic acid or alkyl amidoether carboxylic acid.

An overview of the anionic surfactants used especially in liquid body cleansing compositions can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂₋C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in liquid cleansing compositions according to the invention are nonionic surfactants in admixture with anionic surfactants.

These are described in Schrader, I.c., on pages 600-601 and pp. 694-695. Especially suited are alkyl polyglucosides of the general formula

R₂-O-(R₃O)ₙ-Zₓ,

wherein R₂ is an alkyl group with 8 to 18 carbon atoms, R₃ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from 1 % to 15 %, in particular from 1 % to 10 % by weight, calculated to the total composition.

Mixtures of anionic surfactants and alkyl polyglucosides as well as the use thereof especially in liquid body cleansing compositions are already known, for example, from EP-A 70 074. The alkyl polyglucosides disclosed therein are basically also suited; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from 1 % to 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition.

Such amineoxides are state of the art, for example C₁₂-C₁₈- alkyl dimethyl amineoxides such as lauryl dimethyl amineoxide, C₁₂-C₁₈- alkyl amidopropyl or - ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in liquid cleansing compositions are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As further surfactant component, the compositions can also contain amphoteric or zwitterionic surfactants, for example in an amount from 0.5 % to 15 %, preferably from 1 % to 10 %, by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 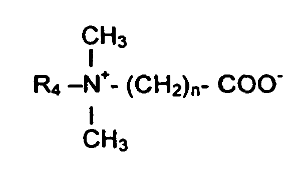 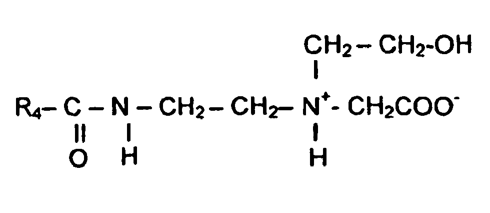 wherein R₄ is a C₈-C₁₈-alkyl group and n is 1 to 3;
sulfobetaines of the structure 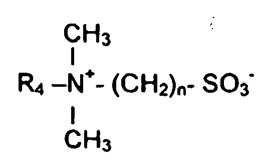 wherein R₄ and n are same as above;
and amidoalkyl betaines of the structure 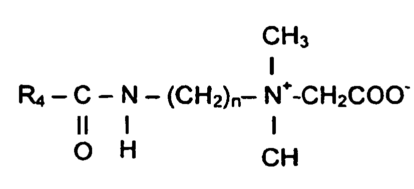 wherein R₄ and n are same as above.

The liquid cleansing composition comprises skin and/or hair conditioning agents. Conditioning agents can be selected from oily substances, non-ionic substances, cationic amphiphilic ingredients, cationic polymers or their mixtures.

Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₅ CO (O CH₂ CH₂)ₙ OH

or

R₅ CO (O CH₂ CH₂)ₙ O OC R₆

where R₅ and R₆ are independent from each other saturated, unsaturated or branched or non-branched alkyl chain with 7 to 21 C atoms and n is typically 2 - 100.

Liquid cleansing compositions, especially designed as personal cleansing compositions comprise further at least one cationic polymer as conditioning agent. Suitable cationic polymers are those of best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quatemium-80, Quatemium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Liquid cleansing compositions may comprise additionally one or more cationic surfactant(s) as conditioner presented with the general formula 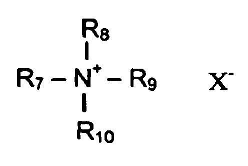 where R₇ is a saturated or unsaturated, branched or non-branched alkyl chain with 8-22 C atoms or

R₁₁ CO NH (CH₂)ₙ

where R₁₁ is saturated or unsaturated, branched or non-branched alkyl chain with 7-21 C atoms and n has value of 1 - 4, or

R₁₂ CO O (CH₂)ₙ

where R₁₂ is saturated or unsaturated, branched or non-branched alkyl chain with 7-21 C atoms and n has value of 1 - 4, and
R₈ is hydrogen or unsaturated or saturated, branched or non-branched alkyl chain with 1 - 4 C atoms or

R₁₁ CO NH (CH₂)ₙ

or

R₁₂ CO O (CH₂)ₙ

where R₁₁, R₁₂ and n are same as above.

R₉ and R₁₀ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimoinium chloride, stearamidopropyl trimonuim chloride, dioleoylethyl dimethyl ammonium methosulfate, dioleoylethyl hydroxyethylmonium methosulfate.

Liquid cleansing compositions may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants should be 0.01 - 5% by weight, preferably 0.01 ― 3.5% by weight, more preferably 0.05 - 2.5% and most preferably 0.1 - 1.5% by weight calculated to the total composition.

Further conditioning additives especially for hair shampoos are hair conditioning and/or styling polymers. These may be nonionic polymers, preferably alcohol-and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful as conditioners in shampoo composition. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

The pH of liquid cleansing compositions is preferably in the range of 2 to 4 and more preferably 2.8 to 3.8.

In principal pH of the compositions can be adjusted with any organic and/or inorganic acids or their mixture. Some of them to mention are phosphoric acid, hydrochloric acid as the inorganic ones and to the organic acids the well known citric acid. Preferred are those of hydroxycarboxylic acids and/or dicarboxylic acids for adjusting pH according to the invention. Accordingly the pH of the compositions is preferably adjusted with hydroxycarboxylic acids and/or dicarboxylic acids. In those cases where selected hydroxycarboxylic acid and/or dicarboxylic acid concentration is not enough to reach the selected pH, other organic and inorganic acids can as well be used to adjust pH to the required value. The hydroxycarboxilic acids useful in the compositions are lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Especially preferred hydroxycarboxylic acids are the lactic and malic acids. Malic acid is also a dicarboxy acid. The most preferred hydroxycarboxylic acid and/or dicarboxylic acid is the malic acid.

Total hydroxycarboxylic acid and/or dicarboxylic acid concentration in the composition varies in the range form 0.1 to 5% by weight, preferably 0.25 to 3% by weight, more preferably 0.5 to 3% by weight and most preferably 0.75 to 3% by weight. In a preferred embodiment of the invention, the compositions of the present invention comprise at least 0.5% malic acid.

Liquid cleansing compositions may be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in liquid cleansing compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

Liquid cleansing composition can comprise organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzylalcohol, benzyloxyethanol and polypropylene glycols. Concentration of organic solvents in the shampoo composition should not exceed 5% by weight, preferably in the range of 0.1 to 3%, more preferably 0.5 to 2.5% by weight calculated to total composition.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

Natural plant extracts are incorporated usually in an amount of 0.01 % to 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4th Ed..

The viscosity of the liquid cleansing compositions according to the invention is in the range of 500 and about 20,000 mPa.s at 20°C, preferably 1,000 to 10,000, in particular 1,000 to 7,000 mPa.s at 20°C, measured with Höppler viscosimeter. Viscosity of compositions can be adjusted with known viscosity enhancers. The preferred ones are glyceryl lauarte, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

The following examples are to illustrate the invention, but not to limit. The compositions are prepared by mixing the individual components in water, whereby it is also possible to use pre-mixtures of various ingredients.

### Measurement of color stability

UV light and sun light stability was determined in a polyethylene terephtalate (PET-P) bottle with a weight of 24.5 ± 0.5 g per 200 ml filling volume by using a UV light irradiation equipment named Sunset Machine model Sunset CPS supplied by Atlas Material Testing Solution. Light emission energy of the equipment is 765 W/m² ± 10%. The bottles used do not contain any UV absorbing substance. Temperature during the measurement was around 46°C ± 2°C. Bottle used had a volume of around 200 ml and a surface area of 0.0108 m² is usually exposed to UV light for 30 h which corresponds to 892 kJ.

In addition to this, the bottles were exposed to direct sunlight on the roof of a building and using a 1 cm² photocell the produced electro energy is measured in Watt. A computing device (counter) -purchased from Kipp & Zonen - Radiation indicator CC20 - reported on the display the energy exposure of the bottled compositions in Wh/m2. The length of the exposure was set at the value 30.000 Wh/m² which is approximately equal to the 892 kJ as in the case of UV light stability measurement. During the tests the same bottles were used as mention in the UV test part.

Finally the color measurements were carried out before and after exposure to UV light or sun light using a Minolta laboratory color measuring device and color differences were recorded. For carrying out the L, a and b measurements, the compositions are first filled into a quartz cuvette (Helma GmbH, Müllheim, Germany) usually used for measuring absorbance of liquids spectrophotmetrically.

The following shampoo composition was used throughout the experimental work for determination of color stability.

**Shampoo Composition**

| | |
|---|---|
| Sodium lauryl ether sulfate | 10.0 (% by wt.) |
| Cocoglucoside | 5.0 |
| Cocoamidopropyl betaine | 1.2 |
| PEG-18 Glyceryl cocoate/oleate | 0.9 |
| Glyceryl laurate | 0.4 |
| Cationic polymer (Polyquatemium-10) | 0.5 |
| Panthenol | 0.1 |
| Sodium chloride | 0.3 |
| Benzylalcohol | 0.5 |
| Malic acid | 0.75 |
| Lactic acid | 0.35 |
| PEG-60-hydrogenated castor oil | 0.5 |
| Fragrance, preservative | q.s. |
| Water | ad 100.0 |

The pH of the composition is 3.7.

The following raw materials are used as surfactants: Sodium lauryl ether sulfate (Texapon N70), Cocoglucoside (Plantacare 818 UP) and Cocoamidopropyl beatine (Tegobetaine F50).

### Example 1

Acid Red 52 was used at a concentration of 0.0006% by weight which was introduced into the composition from a stock solution in water containing 0.1 % acid red 52.

4 composition were examined for their color stability as follows:
**Composition A** not comprising any chelating and UV absorbing agent
**Composition B** comprising only chelating agent EDTA at a concentration of 0.1 % by weight
**Composition C** comprising only UV absorbing agent benzotriazolyl dodecyl p-cresol (Tinogard TL) at a concentration of 0.1 % by weight
**Composition D** comprising both chelating agent EDTA and UV absorbing agent benzotriazolyl dodecyl p-cresol (Tinogard TL) both at a concentration of 0.1 % by weight.

The shampoo composition obtained were exposed to UV light as described above in a 200 ml PET-P bottle. Before and after 30 hrs of exposure, the color measurements, L, a and b values, were carried out as disclosed above with the color measuring device mentioned above. From the L, a and b data ΔE values are calculated using the known formula.

The following results were obtained

| **Composition** | **ΔE** |
|---|---|
| 1-A | 30.6 |
| 1-B | 28.7 |
| 1-C | 29.9 |
| 1-D | 5.8 |

### Example 2

The same as described under Example 1 was carried out using Acid Blue 5 at a concentration of 0.0005% by weight. Same as in the Example 1 the shampoo compositions were exposed to UV light as described above in a 200 ml PET-P bottle. The compositions tested were:
**Composition A** not comprising any chelating and UV absorbing agent
**Composition B** comprising only chelating agent EDTA at a concentration of 0.1 % by weight
**Composition C** comprising only UV absorbing agent benzotriazolyl dodecyl p-cresol (Tinogard TL) at a concentration of 0.1 % by weight
**Composition D** comprising both chelating agent EDTA and UV absorbing agent benzotriazolyl dodecyl p-cresol (Tinogard TL) both at a concentration of 0.1 % by weight.

The following results were obtained

| **Composition** | **ΔE** |
|---|---|
| 2-A | 30.3 |
| 2-B | 30.9 |
| 2-C | 30.4 |
| 2-D | 2.9 |

### Example 3

The same as described under Example 1 was carried out using Basic Red 76 a cationic dyestuff at a concentration of 0.006% by weight. Same as in the Example 1 the shampoo compositions were exposed to UV light as described above in a 200 ml PET-P bottle. The compositions tested were:
**Composition A** not comprising any chelating and UV absorbing agent
**Composition B** comprising only chelating agent EDTA at a concentration of 0.1 % by weight
**Composition C** comprising only UV absorbing agent benzotriazolyl dodecyl p-cresol (Tinogard TL) at a concentration of 0.1 % by weight
**Composition D** comprising both chelating agent EDTA and UV absorbing agent benzotriazolyl dodecyl p-cresol (Tinogard TL) both at a concentration of 0.1% by weight.

The following results were obtained

| **Composition** | **ΔE** |
|---|---|
| 3-A | 34.1 |
| 3-B | 31.4 |
| 3-C | 23.6 |
| 3-D | 0.8 |

Similar results were obtained with the other dyestuffs mentioned in the description.

## Claims

1. Liquid cleansing composition based on at least one surfactant selected from anionic, nonionic, zwitterionic and/or amphoteric ones **characterised in that** it comprises at least one dyestuff selected from cationic, anionic, neutral and plant (natural) dyes, ethylene diamine tetraacetic acid and/or its potassium and sodium salts and/or their mixtures, benzotriazolyl deodecyl p-cresol and has a pH between 2 and 4.

2. Composition according to claim 1 **characterized in that** it comprises at least one anionic and at least one nonionic surfactant.

3. Composition according to claim 2 **characterized in that** it comprises additionally at least one amphoteric and/or zwitterionic surfactant.

4. Composition according to any of the preceding claims **characterized in that** it comprises at least one cationic polymer and/or at least one cationic surfactant as a conditioner.

5. Composition according to any of the preceding claims **characterized in that** it comprises additionally at least one hydroxycarboxylic acid and/or dicarboxylic acid at a concentration of 0.1 to 5% by weight calculated to total Composition.

6. Composition according to claim 5 **characterized in that** it comprises hydroxycarboxylic acid at a concentration of 0.5 to 5% by weight with the condition that it comprises mafic acid at a concentration of not less than 0.5% by weight calculated to total composition.

7. Composition according to any of the preceding claims **characterized in that** it comprises organic solvents at a concentration of less than 5% by weight calculated to total concentration,

8. Composition according to any of the preceding claims **characterized in that** it is a transparent composition,

9. Composition according to any of the claims 1 to 7 **characterized in that** it is a non-transparent pearly composition and contains perlizing agents at a concentration of 0.1 to 3% by weight calculated to total composition.

10. Use of a combination of benzotriazolyl dodecyl p-cresol and ethylenediaminetetraacetic acid and/or its salts for stabilizing the color of liquid cleansing compositions according to any of the preceding claims.

## Patentansprüche

1. Flüssiges Reinigungsmittel, basierend auf mindestens einem Tensid, ausgewählt aus anionischen, kationischen, zwitterionischen und/oder amphoteren Tensiden, **dadurch gekennzeichnet, dass** es mindestens einen Farbstoff, ausgewählt aus kationischen-, anionischen-, neutralen- und Pflanzen-(natürlichen) Farbstoffen, Ethylendiamintetraessigsäure und/oder deren Kalium- und Natriumsalze und/oder deren Mischungen und Benzotriazolyldodecyl p-cresol enthält und einen pH-Wert zwischen 2 und 4 hat.

2. Zusammensetzung nach Anspruch 1, **dadurch gekennzeichnet, dass** sie mindestens ein anionisches und mindestens ein nicht ionisches Tensid enthält.

3. Zusammensetzung nach Anspruch 2, **dadurch gekennzeichnet, dass** sie zusätzlich mindestens ein amphoteres und/oder zwitterionisches Tensid enthält.

4. Zusammensetzung nach einem der vorhergehenden Ansprüche, **dadurch gekennzeichnet, dass** sie mindestens ein kationisches Polymer und/oder mindestens ein kationisches Tensid als einen Konditionierer enthält.

5. Zusammensetzung nach einem der der vorhergehenden Ansprüche, **dadurch gekennzeichnet, dass** sie zusätzlich mindestens eine Hydroxycarboxylsäure und/oder Dicaroxylsäure in einer Menge von 0,1 bis 5 Gew.- %, berechnet auf die Gesamtzusammensetzung, enthält.

6. Zusammensetzung nach Anspruch 5, **dadurch gekennzeichnet, dass** sie Hydroxycarboxylsäure in einer Menge von 0,5 bis 5 Gew.- % enthält, mit der Bedingung, dass sie sie Äpfelsäure in einer Menge von nicht unter 0,5 Gew.-%, berechnet auf die Gesamtzusammensetzung, enthält.

7. Zusammensetzung nach einem der vorhergehenden Ansprüche, **dadurch gekennzeichnet, dass** sie organische Lösungsmittel in einer Menge von weniger als 5 Gew.- %, berechnet auf die Gesamtzusammensetzung, enthält.

8. Zusammensetzung nach einem der vorhergehenden Ansprüche, **dadurch gekennzeichnet, dass** sie eine transparente Zusammensetzung ist.

9. Zusammensetzung nach einem der Ansprüche 1 bis 7, **dadurch gekennzeichnet, dass** sie eine nicht transparente Zusammensetzung mit Perlglanz ist, und Perlglanzmittel in einer Menge von 0,1 bis 3 Gew.- %, berechnet auf die Gesamtzusammensetzung, enthält.

10. Verwendung einer Kombination von Benzotriazolyldodecyl p-cresol und Ethylenediamintetraessigsäure und/oder ihrer Salze zur Stabilisierung der Farbe in flüssigen Reinigungsmitteln nach einem der vorhergehenden Ansprüche.

## Revendications

1. Composition de nettoyage liquide à base d'au moins un agent tensioactif choisi parmi des agents tensioactifs anioniques, non ioniques, zwitterioniques et/ou amphotères **caractérisée en ce qu'**elle comprend au moins un colorant choisi parmi des colorants cationiques, anioniques, neutres et végétaux (naturels), de l'acide éthylènediaminetétraacétique et/ou ses sels de potassium et de sodium et/ou leurs mélanges, du benzotriazolyl dodécyl p-crésol et a un pH entre 2 et 4.

2. Composition selon la revendication 1 **caractérisée en ce qu'**elle comprend au moins un agent tensioactif anionique et au moins un agent tensioactif non ionique.

3. Composition selon la revendication 2 **caractérisée en ce qu'**elle comprend en outre au moins un agent tensioactif amphotère et/ou zwitterionique.

4. Composition selon l'une quelconque des revendications précédentes **caractérisée en ce qu'**elle comprend au moins un polymère cationique et/ou au moins un agent tensioactif cationique comme conditionneur.

5. Composition selon l'une quelconque des revendications précédentes **caractérisée en ce qu'**elle comprend en outre au moins un acide hydroxycarboxylique et/ou un acide dicarboxylique à une concentration de 0,1 à 5% en poids calculée sur la composition totale.

6. Composition selon la revendication 5 **caractérisée en ce qu'**elle comprend un acide hydroxycarboxylique à une concentration de 0,5 à 5% en poids avec la condition qu'elle comprenne de l'acide malique à une concentration de pas moins de 0,5% en poids calculée sur la composition totale.

7. Composition selon l'une quelconque des revendications précédentes **caractérisée en ce qu'**elle comprend des solvants organiques à une concentration de moins de 5% en poids calculée sur la composition totale.

8. Composition selon l'une quelconque des revendications précédentes **caractérisée en ce qu'**il s'agit d'une composition transparente.

9. Composition selon l'une quelconque des revendications 1 à 7 **caractérisée en ce qu'**il s'agit d'une composition nacrée non transparente et qu'elle contient des agents nacrants à une concentration de 0,1 à 3% en poids calculée sur la composition totale.

10. Utilisation d'une combinaison de benzotriazolyl dodécyl p-crésol et d'acide éthylènediaminetétraacétique et/ou de ses sels pour stabiliser la couleur de compositions de nettoyage liquides selon l'une quelconque des revendications précédentes.
